# EUROPEAN PATENT SPECIFICATION

(11) **EP 0 507 580 B1**
(45) Date of publication and mention of the grant of the patent: **31.07.1996**
(21) Application number: 92302899.7
(22) Date of filing: 02.04.1992
(51) Int. Cl.: A61N 1/36

(54) **Implantable apnea generator with ramp on generator**
Implantierbare Vorrichtung mit einem Rampengenerator zur Verhütung des Atemstillstandes
Dispositif implantable comportant un générateur de rampe pour lutter contre l'apnée

(30) Priority: 02.04.1991 US 679120
(43) Date of publication of application: 07.10.1992
(73) Proprietor: MEDTRONIC, INC., Minneapolis, Minnesota 55432-3576 (US)
(72) Inventor: Kallok, Michael, New Brighton, MN 55112 (US); Markowitz, Toby, Roseville, MN 55113 (US)
(74) Representative: Tomlinson, Kerry John

(56) References cited:
- WO-A-91/05583
- DE-A- 2 908 365
- ASAIO TRANSACTIONS vol. 34, no. 3, September 1988, HAGERSTOWN,MD,(US) pages 674 - 680 MICHAEL BRONIATOWSKI ET.AL. 'New Horizons in Dynamic Rehabilitation of Paralyzed Laryngeal Functions'

## Description

The present invention relates generally to implantable medical devices, and more particularly, relates to implantable medical devices for the treatment of obstructive sleep apnea.

The medical characteristics of sleep apnea have been known for some time. There are two generally recognized forms of the disease. The first is central sleep apnea which is associated with the failure of the body to automatically generate the neuro-muscular stimulation necessary to initiate and control a respiratory cycle at the proper time. Work associated with employing electrical stimulation to treat this condition is discussed in "Diaphragm Pacing: Present Status", by William W. L. Glenn, in Pace, Volume I, at pages 357-370 (July - September 1978).

The second condition is known as obstructive sleep apnea. It is discussed at some length in "Obstructive Sleep Apnea: Diagnosis and Treatment", by Drs. Cook and Osguthorpe in Journal of South Carolina Medical Association, 81 (12): 647- 651 (December 1985).

At present, a tracheostomy may be the treatment of choice for a number of patients when obstructive sleep apnea is severe. Some modern commercial systems now employ Continuous Positive Airway Pressure (CPAP). However, interest has recently been displayed in electrical stimulation of the muscle tissue along the upper airway during respiration. U.S. Patent No. 4,830,008 issued to Meer discloses the preamble of claim 1, i.e. a technique for electrical stimulation of the muscles of the upper airway in synchrony with the respiratory cycle. U.S. Patent No. 4,506,666 issued to Durkan discusses such stimulation in conjunction with pressurized airflow supplied by a respirator.

The electrical stimulation of the prior art techniques, however, are primarily concerned with causing contractile motion of the stimulated muscle. This means that the stimulation energy must necessarily be relatively large, and the effects of the stimulation are directly cognizable by the patient.

According to the present invention there is provided an apparatus for the treatment of sleep apnea in a patient comprising
a. determining means for determining the onset of a sleep apnea event;
b. generating means coupled to said determining means for generating a patient stimulation signal in response to said onset of said apnea event; and characterised by:
c. control means coupled to said generating means for varying the amplitude of said patient stimulation signal from a minimum intensity to a maximum intensity.

The present invention overcomes the deficiencies of the prior art by providing a system for treatment of obstructive sleep apnea having a minimum cognizable effect upon the patient. This is particularly important as the purpose of the therapy is to preclude disturbing the patient to ensure that the maximum benefit from sleep is derived.

The system in one embodiment employs at least one sensor to monitor the respiration activity. In this way, the implantable pulse generator can determine the onset of an apnea event from indications within the respiratory cycle of the patient. In the preferred mode, the pressure differential between the thorax and the upper airway is measured. An increase in this differential above a given threshold signifies the onset of an apnea event.

To treat the apnea event, the implantable pulse generator produces a train of pulses which is transferred to the muscles of the upper airway, for example, via an insulated lead and a suitable electrode. The train of pulses is initiated at a relatively low intensity with the pulse intensity increasing over time. In this manner, the patient is provided with a non-cognizable sensation produced by the stimulation signal.

Since effective skeletal muscle stimulation depends upon both the amplitude and frequency of the stimulation pulses, a suitable frequency must be employed. By slowly increasing amplitude, the patient's sensory response to the stimuli exhibits adaptation, a well known phenomenon, that permits the brain to effectively block the pain response to noxious stimuli.

A preferred embodiment of the present invention will now be described, by way of example only, and with reference to the following detailed description and accompanying drawings, in which like reference numerals designate like parts throughout the figures thereof and wherein:
FIG. 1 is a schematic diagram of the respiratory system of a patient;
FIG. 2 is a schematic diagram of the respiratory system showing an obstructive apnea event;
FIG. 3 is a schematic diagram of the respiratory system of a patient having an obstructive apnea treatment system implanted;
FIG. 4 is a graphical representation of the sensor input and implantable pulse generator output of the obstructive apnea treatment system; and,
FIG. 5 is a block diagram of an implantable pulse generator according to an embodiment of the present invention.

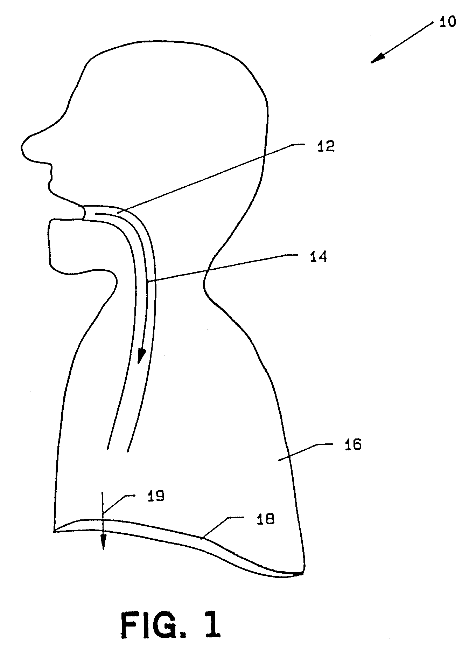

FIG. 1 is a schematic diagram of the respiratory system of patient 10 during inspiration. As a result of the contraction of diaphragm 18 moving in the direction of arrow 19, the volume of thorax 16 is increased. A partial vacuum is created causing air to enter upper airway 12 and proceed in the direction of arrow 14.

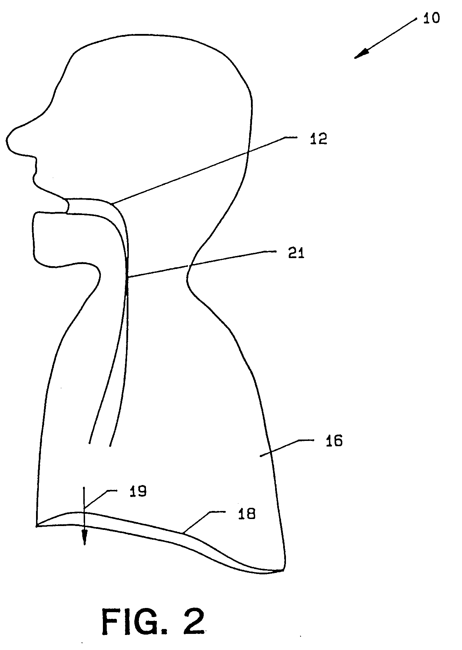

FIG. 2 is a schematic diagram of the respiratory system of patient 10 during an obstructive apnea event. During inspiration, upper airway 12 tends to collapse producing the obstruction to air flow at point 21. The above referenced literature describes in detail the physiological processes associated with the collapse of upper airway 12.

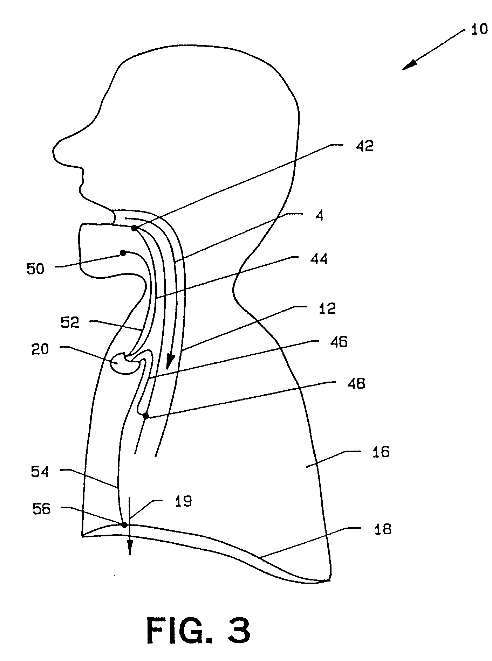

FIG. 3 is a schematic diagram of patient 10 showing implantation of an electrical stimulation system for the treatment of both central and obstructive sleep apnea. Implantable pulse generator 20 is placed subcutaneously at a convenient position. Diaphragm 18 is electrically monitored via electrode 56 coupled to lead 54. This provides the means to synchronize any stimulation supplied to the inspiration cycle.

Patency of upper airway 12 is monitored by pressure sensor 42 and pressure sensor 48 coupled to implantable pulse generator 20 via cables 44 and 46, respectively. Stimulation of the musculature of upper airway 12 is accomplished via lead 52 coupled to electrode 50. All other referenced elements are as previously described.

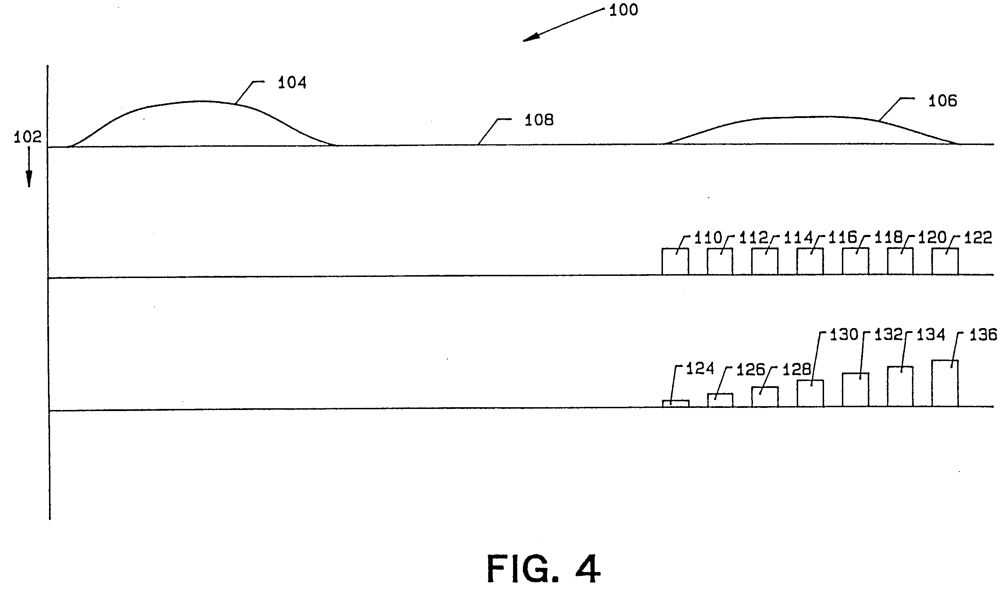

FIG. 4 is a graphical representation 100 of the inputs and outputs of implantable pulse generator 20. Graph 108 represents the pressure differential between the upper airway and the thorax as measured between pressure sensor 42 and pressure sensor 48 wherein a decrease of pressure is shown by arrow 102. Node 104 shows a high pressure difference during inspiration which is indicative of an obstructive apnea event. Node 106 shows a much smaller differential as a result of stimulation of the musculature of the upper airway.

The second graph shows a train of stimulation pulses, including pulses 110, 112, 114, 116, 118, 120, and 122, according to the prior art stimulation technique. Note that each of the pulses is of the same relatively high intensity. This stimulation tends to result in arousal of patient 10 from sleep.

The bottom line shows a train of stimulation pulses generated in accordance with the present invention. First pulse 124 is of minimum intensity. Pulses 126 through 136 are gradually increased in amplitude until maximum intensity is reached with pulse 136. Note that by ramping from low to high intensity it is possible to reach a higher amplitude with less sensation to the patient than is possible with constant intensity stimulation. This permits a more effective contraction with less sensation due to the phenomenon of adaptation. It might also permit one to vary the frequency of stimulation (i.e. reduce frequency) to save energy and prolong the life of the pulse generator.

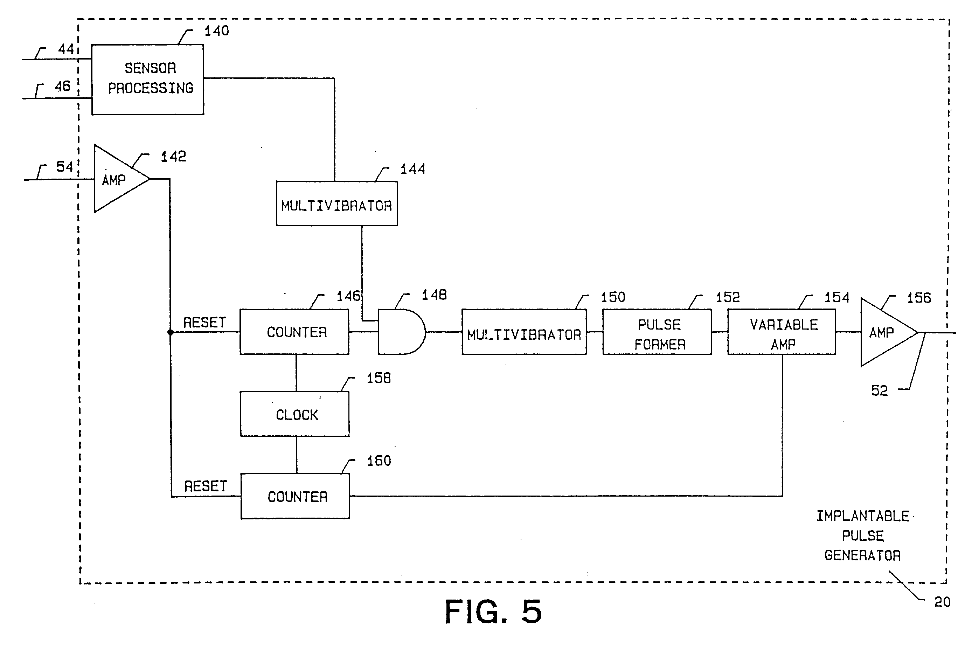

FIG. 5 is a block diagram of implantable pulse generator 20. Sensor processing 140 determines the pressure differential between the upper airway and the thorax by comparing the measurements received from pressure sensors 42 and 48 via cables 44 and 46, respectively. Whenever this difference exceeds a given threshold, an output pulse is supplied to multi-vibrator 144 for pulse shaping.

In particular, a pressure differential above a certain threshold value is identified by sensor processor 140 which outputs a signal to multivibrator 144. Multivibrator 144 then outputs a burst pulse of a predetermined length that determines the length of the desired stimulation pulse at the output 52, although this may vary depending on the operation of counter 146 (see below). After passing through the gate 148, the output signal encounters a second multivibrator 150 which establishes the frequency of the output pulses. The pulses are then shaped by the pulse former 152 into pulses of desired pulse width. Thus, by applying the multivibrator 144, multivibrator 150 and pulse former 152, the duration of the stimulation, the pulse frequency and pulse width may be determined substantially as shown in Fig. 4 pulses 110-122. The variable amplifier then converts these pulses into the ramped pulse forms shown in Fig. 4 pulses 124-136.

The pulse width, pulse frequency and pulse duration parameters desired for the stimulatory pulse burst may be progammed into the device by telemetry signals to the pulse generator (as is conventionally accomplished in heart pacemakers) by implementation of appropriate telemetry circuitry in conjunction with elements 144, 150 and 152.

Each inspiration cycle may be electrically monitored by electrode 56 coupled to lead 54 (see also Fig. 3). The signal is amplified by amplifier 142 and used to reset counter 146 and counter 160. Counter 146 is driven by clock 158 and may be used to delay generation of the stimulation pulse train to synchronize it with the inspiration cycle. And gate 148 provides an output whenever multi-vibrator 144 indicates an obstruction is present as synchronized by counter 146.

The role of sensing the inspiratory stimulus and the clock driven counters is to provide additional control over the timing of the stimulus and the ramping function. The counters 146 and 150 are reset by the start of the sensed activity of the diaphragm at the start of inspiration. Counter 146 may operate in one of two ways.

In a first way, it is set low when reset and is only high after a predetermined number of clock cycles after reset. This may delay the onset of stimulation as stated above depending on the relative timing of the detected apnea. In a second way, it could be set high when reset and only go low after a predetermined number of clock cycles. It could therefore be used to cut off stimulation at a fixed time after the start of sensed inspiration.

Counter 160 may similarly control the amplifier ramping activity. The counter 160 is reset upon sensed inspiration which causes the output to go low. After a predetermined number of clock cycles, the output is set high to begin the ramping of the variable amplifier. By turning on the amplifier before the stimulatory pulses are created, the amplitude of the pulse train starts higher and ends higher than if the amplifier is turned on at the same time the stimulatory pulses begin. Providing the counter is set to go high at the correct time, this will generally occur as activity in the diaphragm will be sensed in advance of a sensed apnea. This provides some control over the amplitude of the stimulatory burst actually delivered to the upper airway.

The counter 160 could also be set to go high as it is reset and then to go low after a predetermined number of clock cycles to turn off the variable amplifier, although this has no immediate physiological applications.

These functions can also be controlled by programming desired values into the counters 146, 160 by telemetry signals to the pulse generator via appropriate telemetry circuitry. The appropriate timing of counters 146, 160 relative to a detected electrical activity in the diaphragm can be determined in use by the physician.

Counter 160 as reset by amplifier 142 controls the gain of variable amplifier 154. In this way, the amplification of the individual pulses output by pulse former 152 is varied to produce the variable intensity output according to the present invention (see also Fig. 4). One skilled in the art will readily appreciate the sort of circuitry required to enable amplifier 154 to provide a steadily increasing amplification upon receipt of a high signal from counter 160. In an alternative embodiment, the counter 160 may provide a stepped output to adjust the gain of the amplifier 154. Amplifier 156 has a fixed gain and supplies the output to lead 52 for transmission to electrode 50 (see also Fig. 3).

Having thus described the preferred embodiments of the present invention, those of skill in the art will be readily able to apply the teachings found herein to yet other embodiments within the scope of the claims hereto attached as interpreted by the description.

## Claims

1. An apparatus for the treatment of sleep apnea in a patient comprising
a. determining means (140) for determining the onset of a sleep apnea event;
b. generating means (144, 150, 152) coupled to said determining means for generating a patient stimulation signal in response to said onset of said apnea event; and characterised by:
c. control means (160, 154) coupled to said generating means for varying over time the amplitude of said patient stimulation signal from a minimum intensity to a maximum intensity.

2. An apparatus according to claim 1 further comprising synchronising means (142, 146) coupled to said generating means for synchronizing said patient stimulation signal with an inspiration cycle.

3. An apparatus according to claim 2 wherein said synchronizing means comprises an electrode for sensing contraction of the diaphragm.

4. An apparatus according to any preceding claim wherein said control means operates to produce said minimum intensity at a beginning of said patient stimulation signal.

5. An apparatus according to any preceding claim wherein said determining means comprises means for measuring a pressure differential between the thorax and the upper airway.

6. An apparatus according to claim 5 wherein said measuring means comprises a first pressure sensor for location in said thorax and a second pressure sensor for location in said upper airway.

## Patentansprüche

1. Vorrichtung zur Behandlung der Schlafapnoe eines Patienten, mit
a) einer Feststellungseinrichtung (140) zum Feststellen des Einsetzens der Schlafapnoe;
b) eine mit der Feststellungseinrichtung verbundene Erzeugungseinrichtung (144, 150, 152) zum Erzeugen eines Patienten-Stimulationssignals in Reaktion auf das Einsetzen der Schlafapnoe; **gekennzeichnet** durch
c) eine mit der Erzeugungseinrichtung verbundene Steuereinrichtung (160, 154) zum Verändern der Amplitude des Patienten-Stimulationssignals mit der Zeit von einer minimalen Intensität zu einer maximalen Intensität.

2. Vorrichtung nach Anspruch 1, mit einer Synchronisationseinrichtung (142, 146), die mit der Erzeugungseinrichtung verbunden ist, um das Patienten-Stimulationssignal mit einem Atemzyklus zu synchronisieren.

3. Vorrichtung nach Anspruch 2, wobei die Synchronisationseinrichtung eine Elektrode zum Erfassen der Kontraktion des Zwerchfells aufweist.

4. Vorrichtung nach einem der vorstehenden Ansprüche, wobei das Steuersystem die minimale Intensität zu Beginn des Patienten-Stimulationssignals erzeugt.

5. Vorrichtung nach einem der vorstehenden Ansprüche, wobei die Festetellungseinrichtung eine Einrichtung zum Messen des Druckunterschieds zwischen dem Brustkorb und dem oberen Atemweg aufweist.

6. Vorrichtung nach Anspruch 5, wobei die Meßeinrichtung einen ersten Drucksensor zum Anbringen im Brustkorb und einen zweiten Drucksensor zum Anbringen im oberen Atemweg aufweist.

## Revendications

1. Dispositif pour le traitement de l'apnée pendant le sommeil chez un patient, comprenant
a. des moyens de détermination (140) pour déterminer le déclenchement d'un phénomène d'apnée pendant le sommeil;
b. des moyens générateurs (144,150,152) couplés auxdits moyens de détermination pour produire un signal de stimulation du patient en réponse audit déclenchement dudit phénomène d'apnée; et
caractérisé par
c. des moyens de commande (160,154) couplés auxdits moyens générateurs pour modifier, dans le temps, l'amplitude dudit signal de stimulation du patient depuis une intensité minimale jusqu'à une intensité maximale.

2. Dispositif selon la revendication 1, comprenant en outre des moyens de synchronisation (142,146) couplés auxdits moyens générateurs pour synchroniser ledit signal de stimulation du patient sur un cycle d'inspiration.

3. Dispositif selon la revendication 2, dans lequel lesdits moyens de synchronisation comprennent une électrode pour détecter une contraction du diaphragme.

4. Dispositif selon l'une quelconque des revendications précédentes, dans lequel lesdits moyens de commande agissent de manière à produire ladite intensité minimale au début dudit signal de stimulation du patient.

5. Dispositif selon l'une quelconque des revendications précédentes, dans lequel lesdits moyens de détermination comprennent des moyens pour mesurer une différence de pression entre le thorax et les voies aériennes supérieures.

6. Dispositif selon la revendication 5, dans lequel lesdits moyens de mesure comprennent un premier capteur de pression destiné à être placé dans ledit thorax et un second capteur de pression destiné à être placé dans lesdites voies aériennes supérieures.
